Europäisches Patentamt

European Patent Office

Office européen des brevets

(11) Publication number: **0 528 033 A1**

# EUROPEAN PATENT APPLICATION
## published in accordance with Art. 158(3) EPC

(21) Application number: 91908860.9

(22) Date of filing: **08.05.91**

(86) International application number:
**PCT/JP91/00611**

(87) International publication number:
**WO 91/17674 (28.11.91 91/27)**

(51) Int. Cl.⁵: **A23L 1/337**

(30) Priority: **11.05.90 JP 122642/90**

(43) Date of publication of application:
**24.02.93 Bulletin 93/08**

(84) Designated Contracting States:
**DE FR GB IT**

(71) Applicant: **YAMANOUCHI PHARMACEUTICAL CO. LTD.**
**No. 3-11 Nihonbashi-Honcho, 2-chome**
**Chuo-ku**
**Tokyo103(JP)**

(72) Inventor: **KIRIYAMA, Shuhachi, Daiya paresu shokubutsuen 803**
**go 1-1, Kita 4-Jo Nishi 12-chome Chuo-ku**
**Sapporo-shi Hokkaido 060(JP)**

(74) Representative: **Geering, Keith Edwin (GB)**
**Reddie & Grose 16 Theobalds Road**
**GB-London WC1X 8PL (GB)**

(54) **WHITE LAVER.**

(57) The invention relates to a white laver obtained by treating a marine alga belonging to the genus Porphyra through the step of decoloration, especially treatment with protease, and the step of treating with an organic solvent or a watery organic solvent; a reddish violet laver obtained in one of the steps for preparing a white laver; a process for preparing a white laver, and so forth. The white laver is widely utilized as a food itself, a component of various food, drink and favorite food, and a drug, while also the reddish violet laver is utilized as a food itself or a component of various food, drink and favorite food.

EP 0 528 033 A1

TECHNICAL FIELD

This invention relates to a white laver to be obtained by processing a seaweed belonging to the genus Porphyra, a method for its preparation, a purplish red laver to be obtained in the method as an intermediate, and a composition containing such a purplish red laver or white laver, etc.

BACKGROUND ART

Laver is an edible seaweed belonging to the division Rhodophycophyta, the genus Porphyra, which has hitherto been taken favorably as food having unique aroma and taste in the East, especially in Japan.

Recently, the techniques of cultivating and processing laver have made progress remarkably, and the yield of laver is increasing year by year. However, the use of laver as food is still limited to, mainly, rolled SUSHI or the like, and the growth rate of consumption of laver is stagnant, which results in a tendency toward overproduction. Accordingly, development of any novel use of laver is a pressing need to promote consumption of laver.

Laver is dark violet, and the dark violet laver is said to be a high quality one in the taste (aroma, etc.) and outward appearance. It is well known that laver contains various pigments, such as green chlorophyll, yellow to reddish violet carotinoids (such as $\beta$-carotene), pink phycoerythrin and bluish violet phycocyanin, and laver is dark violet due to combination of these pigments.

However, where such a blackish food or food component is added to food, drink, or nonessential grocery items, or is used as an additive to cooking materials, it would often spoil the color or beautiful appearance of the food, drink, nonessential grocery items, or cooked products. Therefore, laver has a drawback that its use as food is extremely limited.

On the other hand, dietary fibers have been accepted as important food factors having various physiological functions, which are not inferior to nutrients from dietetic and medical viewpoints. Therefore, increasing interests are taken also in the dietary fibers of seaweeds.

Laver is known to have a cholesterol-lowering effect, and JP-A-58-150515 (the term "JP-A" as used herein means an "unexamined published Japanese patent application") has disclosed a health food containing grains of laver, to lower body cholesterol. In addition, a water-soluble fraction consisting essentially of porphyran, which is obtained from a seaweed belonging to the genus Porphyra, is known to have not only a preventing effect on obesity but also other various preventing effects on the increase of abdominal fats, blood triglyceride, blood cholesterol and blood sugar (refer to JP-A-62-428).

In addition, the present inventor already clarified that laver is effective in the prevention and treatment of hypertriglyceridemia and filed a patent application (refer to Japanese patent Application No. Hei-2-8050).

In order to effectively display such excellent physiological functions of laver, it is desired that laver is added to the food, drink, and nonessential grocery items which can contain a large amount of laver, especially to the staple food and the like which are daily taken in large amounts, and that such foods are continually taken.

However, direct incorporation of laver as it is into daily foods as a food additive was difficult because of the above reasons such as spoiling of beautiful appearance of the foods. In the West, laver is not favored since it is a dark violet food or food component, except other non-blackish seaweeds such as WAKAME (Undaria).

TECHNICAL THEME

Under such circumstances, the present inventor fixed his eyes on overcoming those drawbacks by decoloring or whitening of laver to broaden the use of laver.

No practical demonstration on decoloring or whitening of laver is known up to the present, but only one means of chemically bleaching a different seaweed, i.e., Gracilaria, which is a raw material of agar or FUNORI (a kind of glue), with sodium hypochlorite, sodium hydroxide and chlorine is known (refer to JP-A-50-134900, JP-B-54-6622 - the term "JP-B" as used herein means an "examined Japanese patent publication"). However, this bleaching method decomposes the active ingredients in the seaweed utterly and forms various unknown compounds by the oxidation reaction, which are to be verified to be harmless. Accordingly, this method is not favorable for providing an additive to food and drink as well as to nonessential grocery items and medicines.

DISCLOSURE OF THE INVENTION

Under such a state of art, the present inventor has engaged in the study on a method for removing pigments from laver by any other mild means than chemical bleaching and found that phycobilin pigments such as the aforementioned phycoerythrin and phycocyanin, which would firmly bond to proteins, could not be removed from laver by mere combination of water treatment for removal of water-soluble pigments and organic solvent treatment for removal of fat-soluble pigments. As a result of further studies, the inventor found that a pigment-free white laver can be obtained by processing a seaweed belonging to the genus Porphyra by combination of a step of treatment with a protease and a step of treatment with an organic solvent or a water-containing organic solvent in any desired order. The present invention was accomplished based upon the above findings.

Thus, the present invention relates to a white laver, especially to that obtained by processing a seaweed belonging to the genus Porphyra by the combination of a step of treatment with a protease and a step of treatment with an organic solvent or a water-containing organic solvent in any desired order.

In addition, the present invention relates also to a method of preparation of the said white laver by the combined process of the aforementioned steps in any desired order. Further, the present invention relates also to a purplish red laver obtained as an intermediate on the operation of the preparation method. Furthermore, the present invention relates also to use of such white laver and purplish red laver as food, a composition of food, drink, and nonessential grocery items and a pharmaceutical composition of the white laver.

A white laver of the present invention will be explained in detail hereunder.

The term "white laver" as used herein means the laver obtainable by decoloring of the seaweed belonging to the genus Porphyra. Therefore, it has the same meaning as a "decolored laver" which means the pigment-removed laver. As the case may be, it may be reworded as a "colorless laver" which means the laver having no pigment. Therefore, the term "white laver" as used herein is to be understood to include all of them.

The white laver prepared by the combination of the protease treatment and the organic solvent or water-containing organic solvent treatment shows a pale beige to white color or a pale yellow to white color, though the color differs depending on the order of the treatments combined. Therefore, it is not limited to a sheer white laver. The "white laver" of the present invention is to be understood to include also such a pale beige laver and a pale yellow laver. Where a raw laver is processed to a light purification degree, such a pale beige or yellow color of the processed laver would be somewhat deep, or the processed laver would have a bale grass color or a pale purplish red color. The lavers of these colors are also included within the scope of the present invention. In particular, since the white laver according to the present invention is obtainable by the process of protease treatment and organic solvent or water-containing organic solvent treatment, the dietary fiber content thereof is richer than that of the raw material seaweed while the protein content thereof is less than that of the raw material seaweed. Accordingly, the "white laver" according to the present invention may also be specified by the dietary fiber content and the protein content. Concretely, the dietary fiber content thereof is 50 or more %, preferably 60 or more %; and the protein content thereof is 25 or less %, preferably 20 or less %.

The raw material of a "seaweed belonging to the genus Porphyra" for production of the white laver of the present invention includes all edible seaweeds of a group which is called "laver". Especially, Porphyra tenera KJELLMAN and Porphyra yezoensis VEDA are preferred. The "seaweed belonging to the genus Porphyra" is not limited to a raw laver (raw seaweed) but includes processed laver products from which pigments can be removed by the method according to the present invention. For instance, there are mentioned a so-called "dried laver" (which may also be called a "sheet laver" due to its shape) as obtained by sheeting and drying a raw seaweed; a so-called "roasted laver" as obtained by roasting a dried laver without burning; commercially available "dried laver chips" (which may be crumbled up with hands to be powdery); a "laver powder" as obtained by powdering a dried laver, dried laver chips or a freeze-dried product of a raw laver; and other all processed laver products. Preferred products are a raw laver, a dried laver, dried laver chips and a laver powder.

As the "protease" for the present invention, any and every protease can be used to remove pigments from laver. However, since complete decoloration of laver with endopeptidases such as pepsin or trypsin is difficult, exopeptidases capable of decomposing peptide chains finely are more preferred. In particular, microbial proteases containing both endopeptidases and exopeptidases, which may decompose peptide chains very finely, are especially preferred since they may remove pigments from laver relatively in an extremely short period of time. Concretely, preferable proteases are microbial proteases such as Bacillus licheniformis protease (P-3910, produced by Sigma Co.), P-5380 (produced by Sigma Co.; the name of microorganisms which produces this protease is unknown), as well as microbial proteases derived from Aspergillus oryzae, Aspergillus saitoi, Saccharomyces rouxii, Saccharomyces cerevisiae, Candida krusei, Bacillus subtillis, Streptomyces griseus, Streptococcus lactis, Lactobacillus delbruekii, Lactobacillus brevis, Leuconostoc mesenteroides, and Leuconostoc citrovorum.

Microorganisms which produce protease themselves as well as crude enzymes may be used, but purified proteases are more preferred. Of these enzymes, those extracted from microorganisms which are commercially available for determination of dietary fibers, such as the aforementioned P-3910 and P-5380, are most advantageous for effectively removing pigments from laver.

Organic solvents usable in the treatment process for laver with organic solvent(s) or water-containing organic solvent(s) are all organic solvents which can dissolve out fat-soluble pigments from laver in the treatment process, for example, water-soluble organic solvents of alcohols such as ethanol, propanol or isopropanol, or ketones such as acetone or methyl ethyl ketone; as well as non-polar organic solvents such as dichloromethane, dichloroethane, chloroform, carbon tetrachloride, benzene, toluene or xylene. In particular, water-soluble organic solvents, especially ethanol, are most advantageous for the preparation of the white laver of the present invention.

For preparation of the white laver of the present invention, a seaweed belonging to the genus Porphyra undergoes treatment for decoloring comprising a combination of:

(1) a step of treatment of the seaweed with a protease, and

(2) a step of treatment of the seaweed with an organic solvent or a water-containing organic solvent, in any desired order.

In particular, in the step of treatment of the seaweed with the protease, it is preferred that an aqueous suspension of a raw material seaweed is treated with a protease so that the proteins therein are decomposed and all the water-soluble pigments therein are dissolved out. In the step of treatment of the seaweed with the organic solvent, it is preferred that a raw material seaweed is added to an organic solvent or to a water-containing organic solvent and treated so that the fat-soluble pigments in the raw material seaweed are dissolved out.

Above all, especially advantageous preparation of a white laver of the present invention is a method comprising:

(A) treatment of an aqueous suspension of a seaweed belonging to the genus Porphyra with a protease, and

(B) addition of the treated liquid to a water-soluble organic solvent for the treatment therewith, followed by collection of all the solid precipitated out; or

a method comprising:

(B') addition of a seaweed belonging to the genus Porphyra to an organic solvent or a water-containing organic solvent for the treatment therewith, followed by collection of the solid,

(A) suspension of the obtained purplish red laver in water, followed by treatment of the resulting suspension with a protease, and then

(B) addition of the treated liquid to a water-soluble organic solvent for the treatment therewith, followed by collection of all the solid precipitated out.

The former process comprising the steps (A) and (B) in this order is advantageous since it can remove all the pigments from a raw material seaweed only by the two steps; while the latter process comprising the steps (B'), (A) and (B) in this order is advantageous since it gives an almost completely white laver as the final product. Since the former process comprising the steps (A) and (B) in this order is a modified process from the method of determination of total dietary fibers in food by an enzymatic weight method 985.29 described in Official Methods of Analysis of the Association of Official Analytical Chemists (edited by Kenneth Helrich), 15th Ed., pages 1105 to 1106 (1990), the white laver thus obtained must contain all water-soluble dietary fibers and water-insoluble dietary fibers. Therefore, this process is advantageous also in the point that a white laver having an excellent dietary fiber balance can be obtained.

Next, the process of (A) to (B) and the process of (B') to (A) to (B) will be described in detail hereunder.

The seaweed belonging to the genus Porphyra, which is a raw material to undergo the step (A), is desired to be pulverized as fine as possible in order to conduct the protease treatment efficiently.

For the protease treatment, the raw material seaweed is desired to be incubated under a specific pH condition to maintain and enhance the activity of the protease. The pH value is advantageously a neutral to weak alkaline one ranging from 6 through 8.5, especially from 7 through 8. With the progress of decomposition of proteins with protease, the incubation system becomes acidic. Therefore, dropwise addition of an alkali agent such as ammonium hydroxide or sodium hydroxide for pH adjustment or treatment in a buffer is advantageous. As a buffer usable for this purpose, there are mentioned trishydrochloride buffer as well as other buffers using phosphoric acid, citric acid, amino acids, carbonic acid, boric acid and barbituric acid, etc.

Preferably, the incubation is conducted in the specific temperature range with stirring, so as not to deactivate the protease but to maintain and enhance the activity of the protease in the preferred range. The temperature condition is, though varies depending on the kind of the protease used, but desired to be maintained in a particular range of from 35 through 65°C. For instance, when P-5380 is used as a protease, it is advantageous that the temperature is within the range of from 55 through 65°C, most suitably, 60°C. The aqueous suspension of the raw material seaweed is preferably pre-heated to a particular temperature.

The incubation time is, though varies depending on other various conditions such as the kind of the protease and the temperature, preferably about from 150 through 250 minutes when the incubation is conducted using P-5380 at 60°C.

The object of the present invention may well be attained when the proportion of the seaweed to the protease is approximately from 300/1 through 100/1, but the proportion is not considered to be limited to this proportion. The proportion may be any desired value provided that the protease treatment is conducted efficiently.

By the incubation with a protease, partial decoloration occurs and water-insoluble substances precipitate. Accordingly, the resulting precipitate is taken out by filtration and a water-containing organic solvent may be added thereto. However, as in the following step (B), the incubated liquid may be added directly to an organic solvent (or water-containing organic solvent) gradually, which is rather advantageous in that the filtering operation is required only one time. It is desired that the organic solvent is determined to have such a water content that water-soluble dietary fibers may completely be precipitated out by the addition of the water-soluble organic solvent. In order to precipitate water-soluble dietary fibers completely, the final concentration of the organic solvent may advantageously be set at about 80%.

The precipitation treatment is conducted at room temperature for about 0.5 through 7 days. Static state treatment spending about 24 hours would be sufficient for attainment of the object of the present invention.

The total solid matter thus precipitated out may undergo repeatedly the cycle of (A) to (B), if desired; or they may undergo dialysis, electric osmosis or reverse osmosis with a semipermeable membrane such as cellophane tube, cellulose tube, cellophane membrane, collodion membrane, artificial sulfate paper, natural bladder membrane or air bladder membrane to remove salts derived from the buffer used. Then the solid matter is taken out by the ordinary operation such as filtration.

On the other hand, in the process of (B') to (A) to (B), a seaweed belonging to the genus Porphyra is previously treated with an organic solvent or a water-containing organic solvent to remove components soluble in such a solvent from the seaweed, and the thus pre-treated seaweed is used as the raw material in the following process of (A) to (B). As the organic solvent for the pre-treatment, the same solvents mentioned above may be employed, and ethanol is one of the most preferred solvents in this process.

For the organic solvent treatment, a 100% organic solvent (preferably from 3 through 20 times, most preferably from 5 through 10 times, the amount of the seaweed) is added to a seaweed belonging to the genus Porphyra, preferably to a finely pulverized laver powder, stirring is conducted for several hours at room temperature, and the solid matter is collected.

The water-containing solvent treatment is conducted in such a way that the laver powder as a raw material is added to water, preferably to a desalted water, this is optionally homogenized with a mixer, the resulting aqueous suspension is poured into a 100% organic solvent with stirring, this is allowed to leave statical state for about overnight at room temperature, and the solid matter separated is taken out. The final concentration of the organic solvent in this process is desirably about 80 or more %.

The aforementioned treatments may be repeated until the liquid from which the solid matter has been removed becomes colorless in order to elevate the purity of the product.

According to the aforementioned process, fat-soluble pigments such as chlorophyll and other fat-soluble substances are removed, whereby a brilliant purplish red laver is obtained.

To the aqueous suspension of a purplish red laver obtained in this manner, a protease is added and treatment is conducted in the same manner as in the process of (B) to (A) to obtain a white laver.

In order to elevate the purity of the product, the protease treatment and the organic solvent or water-containing organic solvent treatment may optionally be repeated several times.

The laver from which pigments have almost completely been removed in this way is a powder having a pale beigy white or slightly yellowish white or white color, which contains complete dietary fibers of water-soluble dietary fibers and water-insoluble dietary fibers as well as minerals. It still contains some proteins. In order to decompose the proteins completely, additional protease treatment may be conducted.

On the other hand, the purplish red laver obtained in the step (B') shows a brilliant purplish red color and therefore has an excellent beautiful appearance. In addition, it has another advantage that the purplish red color is stable and does not fade for a long period of time.

The present invention also includes the purplish red laver obtained by the treatment of a seaweed belonging to the genus Porphyra with an organic solvent or a water-containing organic solvent.

The purplish red laver of the present invention is characterized by the method of treatment with an organic solvent or a water-containing organic solvent, and is the laver which is free from fat-soluble components such as chlorophyll which can be dissolved out by treatment with an organic solvent or a water-containing organic solvent.

The treatment with an organic solvent or water-containing organic solvent is already described, and the color of the purplish red laver obtained after this treatment is determined by the color-difference meter analysis to fall within a purple range with, somewhat, a reddish color, as described in the following examples.

INDUSTRIAL APPLICABILITY

The white laver of the present invention contains a lot of dietary fibers and can be used directly as an ordinary food or as a health food or functional food. Furthermore, since it is white, it may be added to various food, drink, and nonessential grocery items as a food additive. In particular, it is useful as a food additive to various food, drink, and nonessential grocery items, without spoiling the color sense or beautiful appearance, or as a colorable food additive. In addition, since it has a fat-depressing function, it is useful also as a medicine for the prevention and treatment of hyperlipidemia and arteriosclerosis.

The white laver and purplish red laver of the present invention are produced in the form of a powder, they may be utilized as powdery foods as they are, or they may also be utilized as a component of a health food, a functional food, or other various food, drink, and nonessential grocery items. The white laver may be used as a medicine in a powder form as it is.

These lavers according to the present invention can be shaped into various forms such as sheets (e.g., sheet lavers), fine grains, granules, tablets, pills, capsules, square form (e.g., solid rouxz), paste (e.g., laver boiled down in soy), and fluid, to obtain solid, semi-solid or fluid foods or medicines.

Laver sheet foods can be produced by any conventional method. For instance, a white laver powder or a purplish red laver powder is uniformly kneaded in a system containing at least one of polyhydric alcohols, glycoalcohols, monoses, bioses or oligosaccharides, optionally together with other dietary fibers, such as carrageenan, alginic acid, sodium alginate, propylene glycol alginate, agar, glucomannan (e.g., KONJAK mannan), guar gum, locust bean gum, tamarind seed gum, xanthane gum, pectin, chitins, pullulane, cyclodextrin, cellulose, cellulose derivatives (e.g., methyl cellulose), as well as proteins or their partial hydrolysates (e.g., laver proteins), soybean proteins, wheat proteins, milk proteins, egg albumen, collagen and microbial proteins; and, if desired, any other food components, for example, soy, seasonings such as sodium glutaminate, spices and flavorings such as sesame, perilla, UME (Japanese apricot), scallion, peanuts, almond, cocoa, coffee, chocolate, pepper, mustard and curry, other seaweeds such as ordinary laver, WAKAME (Undaria), HIJIKI (a kind of brown algae, Hizikia fusiforme) and KOMBU (sea tangle), fishes and shellfishes such as cuttlefish, bonito, tuna, salmon, shrimp, oyster and ASARI (short-necked clam), vegetables such as carrot, cabbage, celery and parsley, fruits such as apple, orange and grapes, vitamins such as vitamin C, lipids such as eicosapentaenoic acid, amino acids such as glutamic acid and alanine, and other additives such as antioxidant and colorant are added to the aqueous solution of the composition. Then, the whole mixture is blended further and then sheeted and dried into sheet laver products.

As the polyhydric alcohols, there are mentioned propylene glycol and glycerin; as the glycoalcohols, there are mentioned sorbitol, maltitol, mannitol and xylitol; as the monoses, there are mentioned glucose, fructose and galactose; as the bioses, there are mentioned sucrose, maltose and lactose; and as the oligosaccharides, there are mentioned galacto-oligosaccharide, fructo-oligosaccharide, isomalto-oligosaccharide, soybean oligosaccharide and KONJAK oligosaccharide.

Where the laver of the present invention is used as a food component of various food, drink, and nonessential grocery items, it is preferably used in such a form that may be added in a large amount, for example, in the form of powder, fine grains or granules. The foods to which the laver of the present invention may be added are not specifically limited. For the purpose of fully attaining the physiological functions of the white laver of the present invention, those which can contain a large amount of white laver or those which would be ingested daily in a large amount are preferable. Concretely, preferable foods are solid or semi-solid foods such as cereals, breads, noodles, pasta, milk products (e.g., butter and cheese), confectionery (e.g., chocolates, rice crackers and cookies), processed marine products, and TOFU (bean curd), as well as fluid foods such as drinks.

The fact that the white laver of the present invention has a fat-depressing effect was clarified by the following tests.

Cholesterol-Lowering Effect of White Laver:

TEST EXAMPLE 1:

Test Method:

As test animals were used SD male rats (6-week age, initial body weight: about 150 g). These rats were fed on a basic diet containing 25% casein as shown in Table 1 below for 8 days. After that, the rats were divided into three groups each consisting of six animals in such a manner that each group might have the same average body weight. One group was continuously fed on the same basic diet for 21 days; while the other two groups were fed on a diet prepared by adding the white laver as obtained in the following Example 4 (6.6%) or cellulose in an equivalent amount as a dietary fiber (5%) to the 25% casein basic diet, respectively, each for 21 days.

Table 1

| Composition of Casein Basic Diet | |
| --- | --- |
| Component | Content (%) |
| Casein | 25 |
| Corn Oil | 5 |
| Mineral Mixture | 4 |
| Vitamin Mixture | 1 |
| Choline Chloride | 0.2 |
| Vitamin E Granules | 0.1 |
| Sucrose | 64.7 |
| Total | 100 |

Test Results:

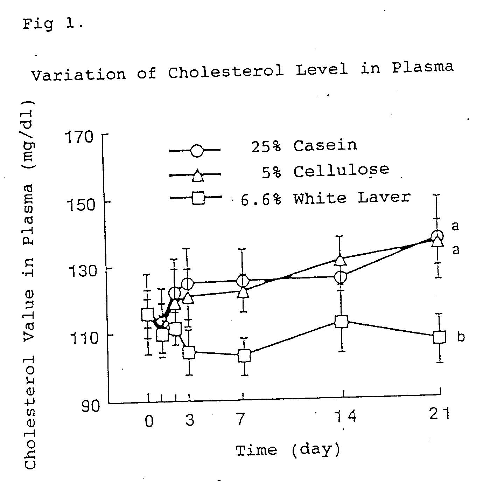

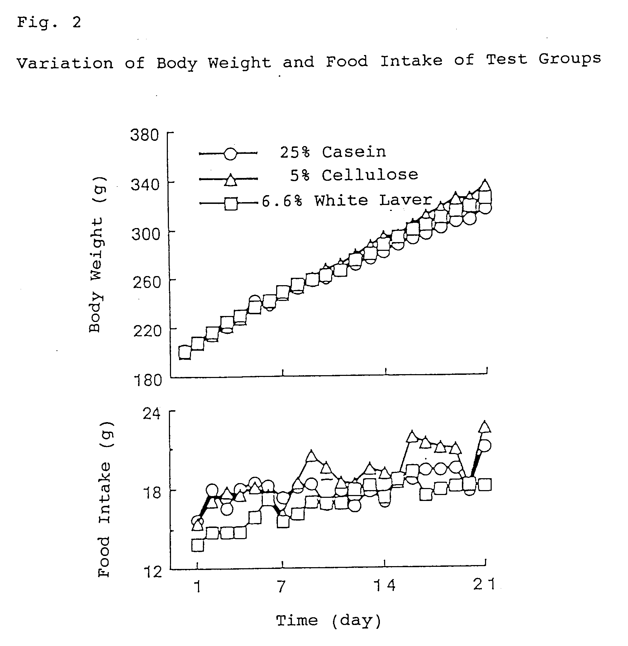

Variation of the cholesterol level in plasma is shown in Fig. 1, and variation of the body weight of each group and variation of the food intake of each group are shown in Fig. 2.

As is shown in Fig. 2, there is no difference in the body weight between the groups, and there is also no significant difference in the food intake between the groups. However, as is shown in Fig. 1, the cholesterol level in plasma significantly lowered in the group which was fed on the white laver-added diet. From the results, it is evident that the white laver containing dietary fibers has higher cholesterol-lowering effect than cellulose.

TEST EXAMPLE 2:

Test Method:

The same test animals were fed for 14 days in the same manner as in Test Example 1, except that a diet prepared by adding 5% white laver prepared in the following Example 6 to the casein basic diet was used.

Test Results:

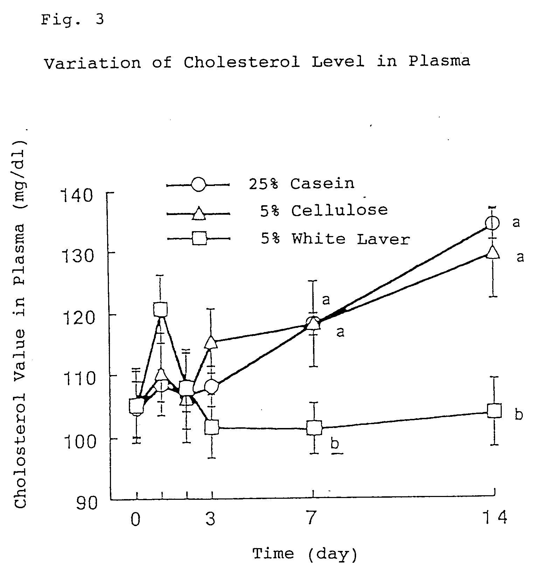

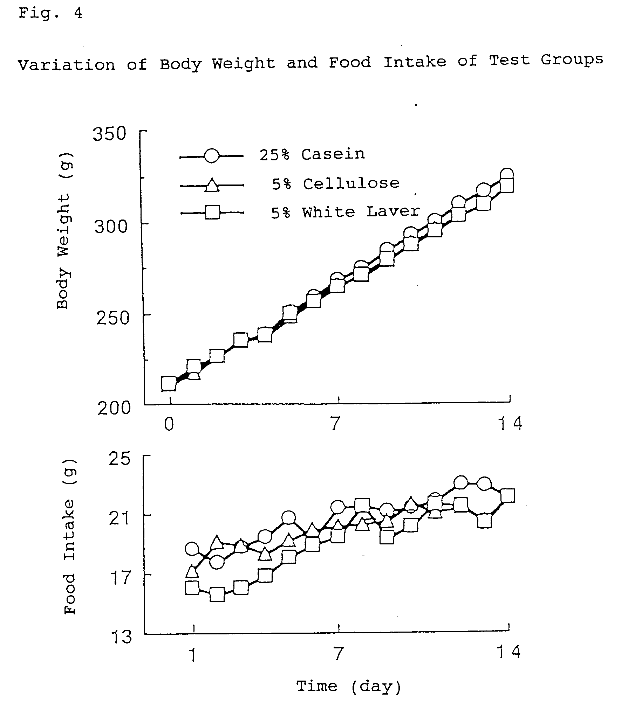

Variation of the cholesterol level in plasma is shown in Fig. 3 and variation of the body weight of each group and variation of the food intake of each group are shown in Fig. 4.

As is shown in Fig. 4, there is no difference in the body weight between the groups, and there is also no significant difference in the food intake between the groups. However, as is shown in Fig. 3, the cholesterol level in plasma significantly lowered in the group which was fed on the white laver-added diet. From the results, it is evident that the white laver containing dietary fibers has higher cholesterol-lowering effect than cellulose.

Furthermore, the same tests were conducted with diets as prepared by adding 5% pectin or 5% chitosan to the casein basic diet. The test results obtained were compared with those of the test animals which were fed on the white laver-added diets. The comparison indicated that the cholesterol-lowering effect of the white laver of the present invention was better.

Accordingly, the white laver of the present invention has a lipid-lowering effect and is useful as a medicine for the prevention and treatment of hyperlipidemia and arteriosclerosis and the related diseases such as cerebral infarction, transient ischemic attack, angina pectoris and peripheral thrombosis and obliterans.

In addition, by its lipid-lowering effect, the white laver of the present invention is also expected to have preventing effect on obesity.

The white laver of the present invention as it is or a mixture of the white laver and any other medicine(s), especially those showing additive or synergistic physiological functions with dietary fibers (e.g., a bifidus bacteria-propagating factor such as oligosaccharides) may be granulated into fine grains or granules by means of various granulating machines or tabletized into tablets or coated tablets by means of a tabletting machine (and a coating machine) and the resulting fine powder, fine grains or granules may optionally be encapsulated into capsules, together with various additives such as vehicle, binder and lubricant, to obtain a pharmaceutical composition of the white laver.

Examples of usable vehicle and binder for this purpose include binders such as starch, gum arabic, gelatin, sorbitol, tragacanth gum, polyvinyl pyrrolidone, etc.; vehicles such as lactose, sugar, corn starch, calcium phosphate, glycine, etc.; and lubricants such as magnesium stearate, talc, polyethylene glycol, silica, etc.

The pharmaceutical composition may be dosed daily through oral administration; and the dose for administration is, though varies depending on the kind of diseases, conditions and ages, about from 0.5 through 50 g/adult a day, preferably about from 1 through 40 g/adult a day, as a white laver powder.

The white laver of the present invention contains well-balanced water-soluble dietary fibers and water-insoluble dietary fibers (e.g., cellulose, hemicellulose, lignin) and is therefore expected to produce more favorable effects. In addition, since it is prepared by mild decoloration treatment of a natural material which has heretofore been taken as food, it is not toxic.

Using the laver of the present invention, health foods and functional foods may be produced and used by adding the aforementioned materials to improve the taste and aroma of foods and other food materials.

BEST MODES FOR CARRYING OUT THE INVENTION

Next, the present invention will be described in detail with reference to examples.

EXAMPLE 1:

Preparation of Laver Powder:

Raw purple laver collected from sea water (grown in Ehime, Japan) was washed with water for several minutes and dewatered at a high speed for several minutes. After dewatering, it was artificially dried at 38 through 40°C for about 2 hours to obtain dried laver chips with a water content of from 12 to 13%. The dried laver chips obtained in this manner were piled to a height of about 5 cm and dried with a hot air keeping a temperature of about 60°C for about 10 to 15 minutes. In order to prevent moisture absorption, the dried laver chips were treated successively in a power mill in which the laver chips were passed through an about 5 cm-mesh screen and in a sample mill in which they were passed through an about 1 mm-mesh screen to obtain a laver powder.

The thus obtained laver powder was packed and preserved in a polyethylene container sealed with a cap equipped with a desiccant.

Preparation of White Laver:

Reagents and Samples:

| (1) | Dried Purple Laver Powder | 1000.35 g |
|---|---|---|
| (2) | Phosphate Buffer (0.05 M; pH 7.4)<br>Sodium Monohydrogen Phosphate 12-Hydrate<br>Sodium Dihydrogen Phosphate Dihydrate<br>Desalted Water | 270 liters<br>261.13 g<br>26.68 g<br>2 liters |

These were adjusted to keep pH value of from 7.4 through 7.5 and then diluted with a desalted water to 18 liters. The operation was repeated 15 times to obtain 270 liters of a buffer.

| (3) | Protease (No. P-5380, product of Sigma Chemical Co., Ltd.) | 5 g |
|---|---|---|

Processing Method:

(i) 200 g of the dried purple laver powder was put into a 15-liter large container, and 10 liters of the phosphate buffer was gently added thereto. The container was maintained in a thermostat bath kept at a temperature of 60°C and the solution therein was stirred for one hour. After the temperature of the solution became 55 through 60°C, a solution of 1 g of the protease No. P-5380 dissolved in 20 ml of the phosphate buffer was added and the solution was incubated for further 3 hours with stirring. During the process, the temperature of the solution was measured at regular intervals of 20 minutes whereby the temperature was confirmed to be within the range from 55°C through 60°C.

40 liters of 100% ethanol was previously put into a 60-liter large container, and the solution thus incubated as above was gradually added thereto with stirring and then it was allowed to stand statically for 24 hours.

The solid as precipitated out was collected by filtration under reduced pressure, and the thus obtained solid was put in 7.5 liters of 80% ethanol, stirred therein for several hours and then filtered. The operation was repeated three times. Then, 7.5 liters of 80% ethanol was added thereto followed by stirring, and the solid formed was taken out by filtration.

(ii) The process (i) was repeated further four times, and 1000.35 g of the purple laver was treated in total. The laver thus treated was collected, shaken for 36 hours, and collected by filtration. The resulting laver was stirred with 7.5 liters of 80% ethanol for several minutes and again collected by filtration. The operation was repeated three times. Then, 7.5 liters of 80% ethanol was added to the laver, and the resulting mixture was stirred, shaken for 6 hours, and allowed to stand statically.

(iii) The solution prepared in the step (ii) was shaken with 6 liters of 100% ethanol and 6 liters of 100% acetone, and then filtered. The solid matter thus obtained was dried with air overnight so that the acetone was completely vaporized out therefrom, to obtain 650.0 g of a pale beige to white laver powder (simple recovery 65%).

Two of the powder sample thus obtained (each weighing 0.5 g) were measured with respect to the protein content by the Kjeldahl method, which showed a protein content of 6.18%. By the analysis of the composition of the raw material purple laver powder, the protein content thereof was determined to be 36.5%. Accordingly, it was clarified that the white laver has a large content of total dietary fibers with still existing some proteins.

EXAMPLE 2:

In accordance with the step (i) of Example 1, 10 g of the dried purple laver powder was put into the container, 400 ml of a desalted water was added thereto, and the whole mixture was heated on a water bath (60°C). The pH value thereof was adjusted to 7.8 with 1N ammonium hydroxide, then a solution of 50 mg of protease No. P-5380 dissolved in 5 ml of 0.08 M phosphate buffer (pH 6.0) was added thereto, and the resulting system was incubated for 2 hours with stirring under heating at 60°C. With progress of the reaction, the pH value of the system lowers. Therefore, the pH value of the system was maintained at about 7.8 by the adjustment with 1 N ammonium hydroxide.

Next, the same treatment as in Example 1 was performed, except that washing was conducted 3 times with 80% ethanol, then 2 times with 100% ethanol and just one time with acetone and that the volume of one batch was adjusted to be about 1 to 2 liters, to obtain a white laver.

The results of the analysis of the white laver are shown in Table 2 below. The color of the laver was, from the result of analysis with a color-difference meter, pale beigy white to white. The dietary fiber content was 63.7%.

EXAMPLE 3:

(1) 300 g of a dried purple laver powder was put into a large container, and 10 liters of a desalted water was added thereto, and the mixture was homogenized with a mixer. This was gradually added to 40 liters of 100% ethanol being stirred with a propeller, little by little, and then allowed to stand statically at room temperature overnight. This ethanol-processed liquid was filtered under suction with a filter paper No. 2 and the resulting laver was washed two times each with 80% ethanol, then just one time with 100% ethanol and more one time with acetone. After drying with air, a brilliant purplish red laver was obtained. The results of the analysis of the purplish red laver are shown in Table 3.

As is clear from the results of analysis, the laver was free from fat-soluble components such as chlorophyll. It was ascertained that the color of this laver does not change even after storage for 7 months at room temperature (results of measurement: L 54.2, a 16.6, b -4.6). It is presumed that these results are attributed to the removal of the unstable chlorophyll and high stability of the remaining pigments.

(2) Using the purplish red laver prepared in this way as a raw material, a white laver was further prepared in the same manner aa in Example 1.

EXAMPLE 4:

A white laver was prepared in the same manner as in Example 1, except that 10 g of a dried purple laver powder was used and that P-5380 was dissolved in 1 ml of 0.05 M phosphate buffer. This white laver was subjected to dialysis against a running water with a cellulose tube, freeze-dried and desalted to obtain a desalted white laver.

The ash content of the white laver thus obtained was 5.9%; while that of the original white laver was 28.1%. The protein content of the former measured by the Kjeldahl method was 7.0%.

The white laver prepared in this way was used in the pharmacological test of the aforementioned Test Example 1.

EXAMPLE 5:

A white laver was obtained in the same manner as in Example 2, except that 0.1 N sodium hydroxide was used and the pH value was adjusted to 7.8 with a pH-stat. The protein content of this white laver measured by the Kjeldahl method was 7.5%, and the ash content thereof was 6.22%.

EXAMPLE 6:

(1) A purplish red laver was obtained in the same manner as in the step (1) of Example 3, except that no desalted water was used but the raw material laver powder was treated with only 100% ethanol and that the washing was conducted three times each with 100% ethanol and just one time with acetone.

The results of analysis of this purplish red laver are shown in Table 3 below. Another purplish red laver prepared in the same manner as this purplish red laver was stored for 2 months at room temperature. The results of analysis thereof are also shown in Table 3. Almost no difference in color was observed.

(2) Using the aforementioned purplish red laver, a white laver was obtained in the same manner as in the step (2) of Example 3.

The results of analysis of this white laver are shown in Table 2 below. As shown in the measurement with a color-difference meter, this white laver shows a slightly yellowish white to white color.

The white laver obtained in this manner was used in the pharmacological test of the aforementioned Test Example 2.

Table 2 - Results of Analysis of White Laver Samples

| Item of Analysis | Raw Material Laver Powder | Example 2 | Example 3 | |
|---|---|---|---|---|
| Water (%) | 5.0 | 9.6 | 10.1 | Hot drying method under normal pressure |
| Proteins (%) | 36.1 | 14.1 | 16.9 | Kjeldahl method (note 1) |
| Lipids (%) | 4.2 | 2.7 | 1.7 | Acid decomposing method |
| Dietary fibers (%) | 39.9<br>42.3 | 63.7 | 64.6 | (note 2)<br>(note 3) |
| Fibers (%) | 7.3 | 2.7 | 1.8 | Improved Henneberg-Stohmann method |
| Ash (%) | 7.5 | 7.2 | 4.9 | Direct firing method |
| Phosphorus (%) | 0.506 | 1.02 | 0.779 | Vanadomolybdic acid absorptiometric method |
| Iron (mg/100 g) | 52.6 | 40.1 | 35.8 | Atomic absorptiometric method |
| Calcium (%) | 0.312 | 0.468 | 0.463 | Atomic absorptiometric method |
| Sodium (%) | 0.125 | 0.540 | 0.554 | Atomic absorptiometric method |
| Potassium (%) | 2.33 | 1.46 | 0.645 | Atomic absorptiometric method |
| Magnesium (mg/100 g) | 222 | 534 | 413 | Atomic absorptiometric method |
| Zinc (ppm) | 72.9 | 66 | 70 | Atomic absorptiometric method |
| Iodine (mg/100 g) | 0.9 (*1) | not detected | not detected | Gaschromatographic method (*1, iodine titration method) |
| Eicosapentaenoic Acid (%) | 1.60 | | | Gaschromatographic method |
| Copper (ppm) | 11.7 | 9 | 13 | Atomic absorptiometric method |
| Manganese (ppm) | 23.0 | 44 | 43 | Atomic absorptiometric method |
| Total chlorophyll (mg/100 g) | 495 | not detected | not detected | Absorptiometric method (visible) |

**EP 0 528 033 A1**

| Item of Analysis | Raw Material Laver Powder | Example 2 | Example 3 | |
|---|---|---|---|---|
| Chlorophyll a (mg/100 g) | | not detected | not detected | |
| Chlorophyll b (mg/100 g) | | not detected | not detected | |
| Color (reflected color) | | | | |
| L | | 70.8 | 77.6 | Color-difference metry |
| a | | -1.8 | 0.8 | |
| b | | 8.8 | 5.5 | |

Note 1: Nitrogen/protein conversion factor, 6.25

Note 2: 100 - (water + proteins + lipids + fibers + ash)

Note 3: By enzyme weight method (AOAC method)

12

Table 3 - Results of Analysis of Purplish Red Laver Samples

| Item of Analysis | Example 3 (1) | Example 6 (1) | After 2 months | |
|---|---|---|---|---|
| Water (%) | 4.8 | 7.4 | 6.2 | Hot drying method under normal pressure |
| Proteins (%) | 40.9 | 40.2 | 42.0 | Kjeldahl method (note 1) |
| Lipids (%) | | 1.6 | 1.8 | Acid decomposing method |
| Dietary fibers (%) | | 41.3 38.3 | 39.9 37.6 | (note 2) (note 3) |
| Fibers (%) | | 3.2 | 3.4 | Improved Henneberg-Stohmann method |
| Ash (%) | | 6.3 | 6.7 | Direct firing method |
| Phosphorus (%) | 0.570 | | | Vanadomolybdic acid absorptiometric method |
| Iron (mg/100 g) | 22.4 | | | Atomic absorptiometric method |
| Calcium (%) | 0.362 | | | Atomic absorptiometric method |
| Sodium (%) | 0.0672 | | | Atomic absorptiometric method |
| Potassium (%) | 1.16 | | | Atomic absorptiometric method |
| Magnesium (mg/100 g) | 293 | | | Atomic absorptiometric method |
| Zinc (ppm) | 92 | | | Atomic absorptiometric method |
| Iodine (mg/100 g) | 2.5 | | | Gaschromatographic method |
| Eicosapentaenoic Acid (%) | not detected | | | Gaschromatographic method |
| Copper (ppm) | 14.9 | | | Atomic absorptiometric method |
| Manganese (ppm) | 29 | | | Atomic absorptiometric method |
| Total chlorophyll (mg/100 g) | not detected | | | Absorptiometric method (visible) |
| Chlorophyll a (mg/100 g) | not detected | | | |

EP 0 528 033 A1

| Item of Analysis | Example 3 (1) | Example 6 (1) | After 2 months | Color-difference metry |
|---|---|---|---|---|
| Chlorophyll b (mg/100 g) | not detected | | | |
| Color (reflected color) | | | | |
| L | 54.0 | 41.2 | 42.4 | |
| a | 17.1 | 10.6 | 11.3 | |
| b | -5.2 | -5.8 | -6.0 | |

Note 1: Nitrogen/protein conversion factor, 6.25

Note 2: 100 − (water + proteins + lipids + fibers + ash)

Note 3: By enzyme weight method (AOAC method)

EXAMPLE 7:

20 parts by weight of the white laver powder obtained in the Example 1 described above and 80 parts by weight of wheat flour were blended, and 3 parts by weight of sugar, 2 parts by weight of edible salt and

14

3 parts by weight of shortening were added thereto, then the whole mixture was kneaded with adding 50 parts by weight of water thereto. A solution of 2 parts by weight of yeast dissolved in 6 parts by weight of water was added. After kneading well, the resulting dough was fermented at about 28°C for 2 hours, degassed and kept up fermentation. This dough was divided into pertinent masses. After proofing, each dough was shaped, put into a mould and baked at 200 through 280°C to obtain bread containing a large amount of white laver.

The color of the bread obtained in this manner was not different from that of the ordinary bread.

EXAMPLE 8:

32 parts by weight of water was added to 160 parts by weight of wheat flour, 4 parts by weight of edible salt and 10 parts by weight of the white laver powder obtained in the Example 1 described above, and the mixture was kneaded. The resulting noodle dough was rolled with a noodling machine and cut into noodles with a cutter. These were dried to obtain white laver-containing noodles. The color of the noodles thus obtained was not different from that of white laver-free noodles.

EXAMPLE 9:

18 parts by weight of water was added to 100 parts by weight of wheat flour, 5 parts by weight of sugar, 10 parts by weight of shortening, 20 parts by weight of the white laver powder obtained in Example 1, 0.4 parts by weight of sodium bicarbonate, 0.6 parts by weight of ammonium carbonate and 0.5 parts by weight of edible salt, and the mixture was kneaded, shaped and baked at 150 through 250°C to obtain white-laver-containing crackers. The color of these crackers was not different from that of the ordinary crackers.

EXAMPLE 10:

3 parts by weight of edible salt was added to 100 parts by weight of minced tuna, and the mixture was kneaded. To the resulting mixture were added 30 parts by weight of the white laver powder as obtained in Example 1, 1 part by weight of sugar and small amounts of glutamic acid, starch, spices, antiseptics, pyrolignous acid, vitamin A, etc. Further, shortening oil was added thereto, and the resulting mixture was put into a casing and sealed, and sterilized under heat by soaking in a hot water of from 85 through 88°C for 50 minutes. After cooling, white laver-containing fish sausages were obtained. These sausages were somewhat more whitish than the ordinary sausages.

EXAMPLE 11:

10 parts by weight of the white laver powder of Example 1 was added to 100 parts by weight of durum flour, a small amount of egg and from 3 through 4 parts by weight of edible salt, and the mixture was stirred and kneaded with adding water to obtain a dough. This dough was appropriately rolled out to obtain a paste having a water content of about 30%. The paste as such or stuffed with meats, cheese, vegetables, etc., was heated at about 100°C for 2 minutes and packaged to obtain a preservable paste product, the color of which was not different from that of the ordinary paste product.

EXAMPLE 12:

90 parts by weight of the white laver powder of Example 1 and 10 parts by weight of soybean hydrolysate were kneaded with 25 parts by weight of 70% aqueous solution of maltose to obtain a powdery composition. 1.05 liters of water was added to this composition. After blending, the resulting paste was sheeted by the wet casting method to obtain white sheet lavers.

EXAMPLE 13:

To 95 parts by weight of the white laver powder obtained in Example 1 described above was added the same amount of 5% aqueous solution of corn starch, and the mixture was kneaded with a kneader. The resulting mixture was granulated with an extrusion-granulating machine into pillar granules. These granules were sieved and the resulting uniform granules having a mean grain size suitable for a granular preparation were dried with a fluidizing drier to obtain white laver granules.

15

EP 0 528 033 A1

BRIEF EXPLANATION OF THE DRAWINGS

Fig. 1 shows variation of cholesterol level in plasma in the pharmacological test (Test Example 1) for the lipid-lowering effect of the white laver obtained in Example 4.

Fig. 2 shows variation of the body weight and the food intake of each group in Test Example 1.

Fig. 3 shows variation of cholesterol level in plasma in the pharmacological test (Test Example 2) for the lipid-lowering effect of the white laver obtained in Example 6.

Fig. 4 shows variation of the body weight and the food intake of each group in Test Example 2.

**Claims**

1. A white laver obtained by a process comprising decoloring a seaweed belonging to the genus Porphyra.

2. The white laver as claimed in claim 1 having a high content of dietary fibers, which is obtained by a decoloration of a seaweed belonging to the genus Porphyra which comprises a step of treatment with a protease and a step of treatment with an organic solvent or a water-containing organic solvent in any desired order.

3. The white laver as claimed in claim 2, which is obtained by a process comprising treating an aqueous suspension of a seaweed belonging to the genus Porphyra with a protease, adding the treated liquid to a water-soluble organic solvent, and collecting all the solids formed.

4. The white laver as claimed in claim 3, which is obtained by a process comprising treating a seaweed belonging to the genus Porphyra with an organic solvent to decolor a part of the pigments therein, treating an aqueous suspension of the resulting purplish red laver with a protease, adding the treated liquid to a water-soluble organic solvent, and collecting all the solids formed.

5. The white laver as claimed in any of claims 1 to 4, which has a dietary fiber content of 50 or more %.

6. The white laver as claimed in claim 5, which has a dietary fiber content of 50 or more % and a protein content of 25 or less %.

7. A purplish red laver obtained by a process comprising treating a seaweed belonging to the genus Porphyra with an organic solvent or a water-containing organic solvent to decolor a part of the pigments therein.

8. A method for preparing a white laver, which comprises a decoloration of a seaweed belonging to the genus Porphyra which comprises a step of treatment with a protease and a step of treatment with an organic solvent or a water-containing organic solvent in any desired order.

9. The method as claimed in claim 8, which comprises treating an aqueous suspension of a seaweed belonging to the genus Porphyra with a protease, adding the treated liquid to a water-soluble organic solvent, and collecting all the solids formed.

10. The method as claimed in claim 9, which comprises treating a seaweed belonging to the genus Porphyra with an organic solvent to decolor a part of the pigments therein, treating an aqueous suspension of the resulting purplish red laver with a protease, adding the treated liquid to a water-soluble organic solvent, and collecting all the solids formed.

11. A pharmaceutical composition comprising a pharmacologically effective amount of the white laver as claimed in claim 1 and a pharmaceutically acceptable carrier.

12. The pharmaceutical composition as claimed in claim 11, which is a lipid-lowering agent.

13. A method for treating hyperlipidemia or arteriosclerosis, which comprises administrating the pharmaceutical composition as claimed in claim 11 to a patient in an effective amount for treatment.

16

**14.** Use of the white laver as claimed in claim 1 as food.

**15.** Use of the purplish red laver as claimed in claim 7 as food.

**16.** Food, drink, or nonessential grocery items containing the white laver as claimed in claim 1.

**17.** Food, drink, or nonessential grocery items containing the purplish red laver as claimed in claim 7.

Fig 1.

Variation of Cholesterol Level in Plasma

Fig. 2

Variation of Body Weight and Food Intake of Test Groups

Fig. 3

Variation of Cholesterol Level in Plasma

Fig. 4

Variation of Body Weight and Food Intake of Test Groups

## INTERNATIONAL SEARCH REPORT

International Application No PCT/JP91/00611

### I. CLASSIFICATION OF SUBJECT MATTER (if several classification symbols apply, indicate all) 6

According to International Patent Classification (IPC) or to both National Classification and IPC

Int. Cl$^5$ A23L1/337

### II. FIELDS SEARCHED

Minimum Documentation Searched 7

| Classification System | Classification Symbols |
|---|---|
| IPC | A23L1/337 |

Documentation Searched other than Minimum Documentation
to the Extent that such Documents are Included in the Fields Searched 8

### III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | JP, B1, 54-21427 (Kyowa Hakko Kogyo Co., Ltd.), July 30, 1979 (30. 07. 79), (Family: none) | 1-6, 8-14, 16 |
| A | JP, C2, 155703 (Tomokichi Tsuda), April 19, 1943 (19. 04. 43), Claim (Family: none) | 1-17 |
| A | JP, C2, 135922 (Michiyuki Akiyama, Kosuke Katakura, Sadagoro Handa), June 7, 1940 (07. 06. 40), Claim (Family: none) | 1-17 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

### IV. CERTIFICATION

| Date of the Actual Completion of the International Search | Date of Mailing of this International Search Report |
|---|---|
| July 23, 1991 (23. 07. 91) | August 12, 1991 (12. 08. 91) |

| International Searching Authority | Signature of Authorized Officer |
|---|---|
| Japanese Patent Office | |

Form PCT/ISA/210 (second sheet) (January 1985)